(19) Europäisches Patentamt
European Patent Office
Office européen des brevets

(11) EP 4 252 756 A1

(12) EUROPEAN PATENT APPLICATION
published in accordance with Art. 153(4) EPC

(43) Date of publication:
**04.10.2023 Bulletin 2023/40**

(21) Application number: **21898030.8**

(22) Date of filing: **25.11.2021**

(51) International Patent Classification (IPC):
***A61K 31/683*** *(2006.01)* ***A61P 11/00*** *(2006.01)*
***A61P 43/00*** *(2006.01)*

(52) Cooperative Patent Classification (CPC):
**A61K 31/683; A61P 11/00; A61P 43/00**

(86) International application number:
**PCT/JP2021/043186**

(87) International publication number:
**WO 2022/114058 (02.06.2022 Gazette 2022/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.11.2020 JP 2020195818**

(71) Applicant: **Sansho Co., Ltd.**
**Tokyo 103-0027 (JP)**

(72) Inventors:
- **SUZUKI Kumiko**
  **Chiba-shi, Chiba 262-0026 (JP)**
- **KOBAYASHI Katsutoshi**
  **Tsukuba-shi, Ibaraki 305-0029 (JP)**
- **IMAMURA Shigeyuki**
  **Izunokuni-shi, Shizuoka 410-2321 (JP)**
- **MOROHOSHI Toshiro**
  **Isehara-shi, Kanagawa 259-1133 (JP)**

(74) Representative: **Godemeyer Blum Lenze Patentanwälte Partnerschaft mbB - werkpatent**
**An den Gärten 7**
**51491 Overath (DE)**

# (54) THERAPEUTIC AGENT FOR PULMONARY FIBROSIS

(57) An object of the present invention is to provide a therapeutic agent for pulmonary fibrosis which has high therapeutic effect. The invention provides a therapeutic agent for pulmonary fibrosis which comprises, as an active ingredient, a compound represented by the following formula (I):

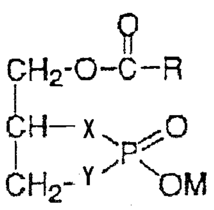

(I)

wherein R represents a linear or branched alkyl group having 1 to 30 carbon atoms, a linear or branched alkenyl group having 2 to 30 carbon atoms, or a linear or branched alkynyl group having 2 to 30 carbon atoms, which may contain a cycloalkane or aromatic ring; X and Y each independently represent an oxygen atom or a methylene group, provide that X and Y do not simultaneously represent a methylene group; and M represents a hydrogen atom or an alkali metal atom.

[Fig. 2]

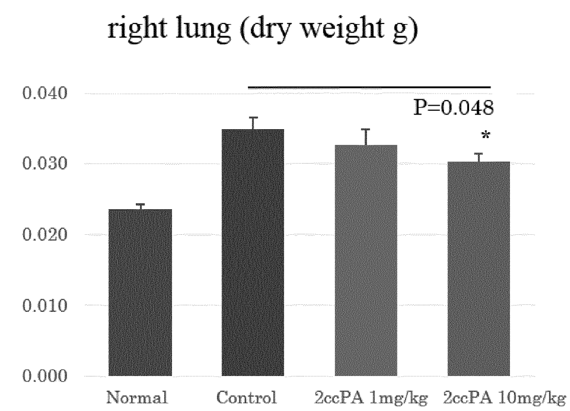


EP 4 252 756 A1

## Description

Technical Field

The present invention relates to a therapeutic agent for pulmonary fibrosis comprising, as an active ingredient, cyclic phosphatidic acid, carbacyclic phosphatidic acid or crystal form thereof.

Background Art

Pulmonary fibrosis is a manifestation of fibrosis of lung tissue due to accumulation of excess collagen and other extracellular matrix. Among pulmonary fibrosis, idiopathic pulmonary fibrosis is a chronic refractory disease with a poor prognosis, with an average intermediate survival of 3 years and a 5-year survival rate of 20-40%. Despite the poor prognosis of idiopathic pulmonary fibrosis, there are few drug treatments that should be recommended. Traditionally, steroids and immunosuppressive drugs have been used tentatively, but in recent years, evidence has accumulated that these drugs worsen prognosis and these are no longer recommended treatments. Antifibrotic agents such as pirfenidone and nintedanib have been introduced to the market after scientific validation studies and are beginning to be used as novel therapies, but there is a need to develop more effective therapies.

The inventors have found that certain cyclic phosphatidic acid derivatives and salts thereof have excellent preventive and therapeutic effects on arthritis such as rheumatoid arthritis and osteoarthritis (Patent Document 1), atopic dermatitis (Patent Document 2), and inhibition of cancer metastasis and invasion (Patent Document 3).

Prior Art Documents

Patent Documents

Patent Document 1: Japanese Patent No. 5465815
Patent Document 2: Japanese Patent Publication No. 2012-056853
Patent Document 3: Japanese Patent No. 4163007

Summary of the Invention

Object to Be Attained by the Invention

As described above, it was known that certain cyclic phosphatidic acid derivatives and salts thereof exhibit certain pharmacological effects, but it was not known that cyclic phosphatidic acid derivatives and salts thereof have therapeutic effects on pulmonary fibrosis.

An object of the present invention is to provide a therapeutic agent for pulmonary fibrosis which has high therapeutic effect.

Means for Attaining the Object

As a result of diligent study to solve the above object, the present inventors have discovered that cyclic phosphatidic acid and its derivatives are effective in treating pulmonary fibrosis and have completed the present invention.

Thus, the present invention provides the following invention.

<1> A therapeutic agent for pulmonary fibrosis which comprises, as an active ingredient, a compound represented by the following formula (I):

[Formula 1]

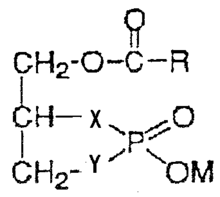

(I)

wherein R represents a linear or branched alkyl group having 1 to 30 carbon atoms, a linear or branched alkenyl group having 2 to 30 carbon atoms, or a linear or branched alkynyl group having 2 to 30 carbon atoms, which may contain a cycloalkane or aromatic ring; X and Y each independently represent an oxygen atom or a methylene group, provide that X and Y do not simultaneously represent a methylene group; and M represents a hydrogen atom or an alkali metal atom.

<2> The therapeutic agent for pulmonary fibrosis according to <1>, wherein, in Formula (I), either X or Y represents an oxygen atom and the other represents a methylene group.

<3> The therapeutic agent for pulmonary fibrosis according to <1> or <2>, wherein the compound represented by Formula (I) is carbacyclic phosphatidic acid of 1-oleoyl-cyclic phosphatidic acid, 1-palmitoleoyl-cyclic phosphatidic acid, or a derivative thereof.

The present invention further provides a method for treatment of pulmonary fibrosis comprising administering a compound represented by the aforementioned Formula (I) to a patient with pulmonary fibrosis.

The present invention further provides use of a compound represented by the aforementioned Formula (I) for production of a therapeutic agent for pulmonary fibrosis.

Effects of the Invention

The present invention can provide a therapeutic agent for pulmonary fibrosis which has high therapeutic effect.

Brief Description of the Drawings

[Fig. 1] Fig. 1 shows the effect of 2ccPA on the wet weight of the right lung of bleomycin-induced pulmonary fibrosis model mice.

[Fig.2] Fig.2 shows the effect of 2ccPA on the dry weight of the right lung of bleomycin-induced pulmonary fibrosis model mice.

[Fig.3] Fig. 3 shows the effect of 2ccPA on hydroxyproline in the right lung of bleomycin-induced pulmonary fibrosis model mice.

Embodiments for Carrying out the Invention

Hereafter, the present invention is described in greater detail.

The therapeutic agent for pulmonary fibrosis according to the present invention can be used for treatment of pulmonary fibrosis. Diseases that cause fibrosis of the lungs include interstitial pneumonia, cystic fibrosis, chronic obstructive pulmonary disease (COPD), acute respiratory distress syndrome (ARDS), inflammatory lung diseases, lung infections, radiological pneumonia, drug-induced interstitial pneumonia, and interstitial pneumonia associated with collagen diseases. Idiopathic pulmonary fibrosis is especially preferred among idiopathic interstitial pneumonias of which the cause is unknown. Idiopathic interstitial pneumonias (IIPs) include the following clinicopathologic diseases: idiopathic pulmonary fibrosis (IPF), nonspecific interstitial pneumonia (NSIP), idiopathic organizing pneumonia (COP/BOOP), acute interstitial pneumonia (AIP), exfoliative interstitial pneumonia (DIP), and interstitial lung disease associated with respiratory bronchiolitis (RB-ILD), Lymphocytic interstitial pneumonia (LIP), etc.

The therapeutic agent for pulmonary fibrosis according to the present invention comprises, as an active ingredient, cyclic phosphatidic acid, carbacyclic phosphatidic acid, or a salt thereof. Any compound can be used as cyclic phosphatidic acid, carbacyclic phosphatidic acid, or a salt thereof without particular limitation, provided that such compound exhibits the effects of the present invention. Preferable examples include compounds represented by formula (I) below:

[Formula 2]

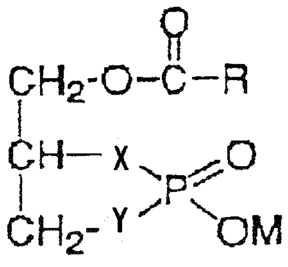

(I)

wherein R represents a linear or branched alkyl group having 1 to 30 carbon atoms, a linear or branched alkenyl group having 2 to 30 carbon atoms, or a linear or branched alkynyl group having 2 to 30 carbon atoms, which may contain a cycloalkane or aromatic ring; X and Y each independently represent an oxygen atom or a methylene group, provided that X and Y do not simultaneously represent a methylene group; and M represents a hydrogen atom or an alkali metal atom.

In Formula (I), specific examples of a linear or branched alkyl group having 1 to 30 carbon atoms represented by a substituent R include a methyl group, an ethyl group, a propyl group, a butyl group, a pentyl group, a hexyl group, a heptyl group, an octyl group, a nonyl group, a decyl group, a undecyl group, a dodecyl group, a tridecyl group, a tetradecyl group, a pentadecyl group, a hexadecyl group, a heptadecyl group, an octadecyl group, a nonadecyl group, and an eicosyl group.

Specific examples of linear or branched alkenyl groups having 2 to 30 carbon atoms represented by a substituent R include an allyl group, a butenyl group, an octenyl group, a decenyl group, a dodecadienyl group, and a hexadecatrienyl group. More specific examples thereof include an 8-decenyl group, an 8-undecenyl group, an 8-dodecenyl group, an 8-tridecenyl group, an 8-tetradecenyl group, an 8-pentadecenyl group, an 8-hexadecenyl group, an 8-heptadecenyl group, an 8-octadecenyl group, an 8-icocenyl group, an 8-docosenyl group, a heptadeca-8,11-dienyl group, a heptadeca-8,11,14-trienyl group, a nonadeca-4,7,10,13-tetrenyl group, a nonadeca-4,7,10,13,16-pentenyl group, and a henicosa-3,6,9,12, 15, 18-hexenyl group.

Specific examples of linear or branched alkynyl groups having 2 to 30 carbon atoms represented by a substituent R include an 8-decynyl group, an 8-undecynyl group, an 8-dodecynyl group, an 8-tridecynyl group, an 8-tetradecynyl group, an 8-pentadecynyl group, an 8-hexadecynyl group, an 8-heptadecynyl group, an 8-octadecynyl group, an 8-icocynyl group, an 8-dococynyl group, and a heptadeca-8,11-diynyl group.

Specific examples of the cycloalkane ring, which may be contained in the above-described alkyl, alkenyl, or alkynyl group, include a cyclopropane ring, a cyclobutane ring, a cyclopentane ring, a cyclohexane ring, and a cyclooctane ring. The cycloalkane ring may contain one or more hetero atoms, and examples thereof include an oxylane ring, an oxetane ring, a tetrahydrofuran ring, and an N-methylprolidine ring.

Specific examples of the aromatic ring, which may be contained in the above-described alkyl, alkenyl, or alkynyl group, include a benzene ring, a naphthalene ring, a pyridine ring, a furan ring, and a thiophene ring.

When the substituent R is an alkyl group substituted with a cycloalkane ring, accordingly, specific examples include a cyclopropylmethyl group, a cyclohexylethyl group, and an 8,9-methanopentadecyl group.

When the substituent R is an alkyl group substituted with an aromatic ring, specific examples include a benzyl group, a phenetyl group, and a p-pentylphenyloctyl group.

Preferably, R represents a linear or branched alkyl group having 9 to 17 carbon atoms, a linear or branched alkenyl group having 9 to 17 carbon atoms, or a linear or branched alkynyl group having 9 to 17 carbon atoms. More preferably, R represents a linear or branched alkyl group having 9, 11, 13, 15, or 17 carbon atoms or a linear or branched alkenyl group having 9, 11, 13, 15, or 17 carbon atoms. Particularly preferably, R represents a linear or branched alkenyl group having 9, 11, 13, 15, or 17 carbon atoms.

X and Y in the compound represented by Formula (I) each independently represent an oxygen atom (-O-) or a methylene group ($-CH_2-$), provided that X and Y do not simultaneously represent a methylene group. That is, combinations of X and Y include the following three patterns:

(1) X represents an oxygen atom and Y represents an oxygen atom;
(2) X represents an oxygen atom and Y represents a methylene group; or
(3) X represents a methylene group and Y represents an oxygen atom.

M in the cyclic phosphatidic acid derivative represented by Formula (I) represents a hydrogen atom or an alkali metal atom. Examples of alkali metal atoms include lithium, sodium and potassium, with sodium being particularly

preferable.

Specifically, the compound represented by Formula (I) in the present invention is particularly preferably a cyclic phosphatidic acid or a carbacyclic phosphatidic acid derivative having, as an acryl group at position 1, an oleoyl group in which the substituent R represents an alkenyl group having 17 carbon atoms (abbreviated as "C18:1") or a palmitoleoyl group in which the substituent R represents an alkenyl group having 15 carbon atoms (abbreviated as "C16:1").

The compound represented by Formula (I) may comprise an isomer, such as a positional isomer, geometric isomer, tautomer, or optical isomer, in accordance with the type of a substituent thereof. All possible isomers and mixtures comprising two or more types of such isomers at a certain ratio are within the scope of the present invention.

In addition, the compound represented by Formula (I) may be in the form of an adduct composed of the compound and water or various types of solvents (hydrates or solvates). Such adduct is also within the scope of the present invention. Moreover, any crystal forms of the compound represented by Formula (I) and salts thereof are also within the scope of the present invention.

The crystal form of the compound represented by Formula (1) includes, for example, crystals of cyclic phosphonic acid sodium salt as described in Japanese Patent No. 6736466. In Japanese Patent No. 6736466, it is described that crystals of cyclic phosphonic acid sodium salt can be produced by a step of reacting cyclic phosphonate with sodium halide in an organic solvent to obtain 2ccPA, and a step of precipitating crystals by concentrating the solution containing 2ccPA obtained in the above step under reduced pressure or by cooling the solution containing 2ccPA obtained in the above step. The crystal form of the compound represented by Formula (1) may be produced by the method described in Japanese Patent No. 6736466, or by a method other than the above.

A compound represented by Formula (I) in which both X and Y represent oxygen atoms can be chemically synthesized in accordance with the method described in, for example, JP Patent Publication (Kokai) No. H05-230088 A (1993), H07-149772 A (1995), H07-258278 A (1995), or H09-25235 A (1997).

Also, a compound represented by Formula (I) in which both X and Y represent oxygen atoms can be synthesized in accordance with the method described in JP Patent Publication (Kokai) No. 2001-178489 A by allowing phospholipase D to react with lysophospholipid. Lysophospholipid used herein is not particularly limited, so long as it is capable of reacting with phospholipase D. Many types of lysophospholipids are known, molecular species having different types of fatty acids or having ether or vinyl ether bonds are known, and such lysophospholipids are commercially available. As phospholipase D, those derived from higher-order plants such as cabbage or peanuts or those derived from microorganisms such as *Streptomyces chromofuscus* or *Actinomadula sp.,* are commercially available as reagents, although a cyclic phosphatidic acid is synthesized by an enzyme derived from the *Actinomadula sp.* No. 362 strain in a very selective manner (JP Patent Publication (Kokai) No. H11-367032 A (1999)). The reaction between lysophospholipid and phospholipase D may be carried out under any conditions, so long as an enzyme is able to exert its activity. For example, the reaction can be carried out in an acetate buffer containing calcium chloride (pH: about 5 to 6) at room temperature or higher (preferably 37°C) for 1 to 5 hours. The resulting cyclic phosphatidic acid derivative can be purified in accordance with a conventional technique by means of, for example, extraction, column chromatography, or thin-layer chromatography (TLC).

A compound represented by Formula (I) in which X represents an oxygen atom and Y represents a methylene group can be synthesized in accordance with the method described in literature (Kobayashi, S. et al., Tetrahedron Letters, 34, 4047-4050, 1993) or WO 2002/094286. An example of a specific synthetic pathway is shown below.

[Formula 3]

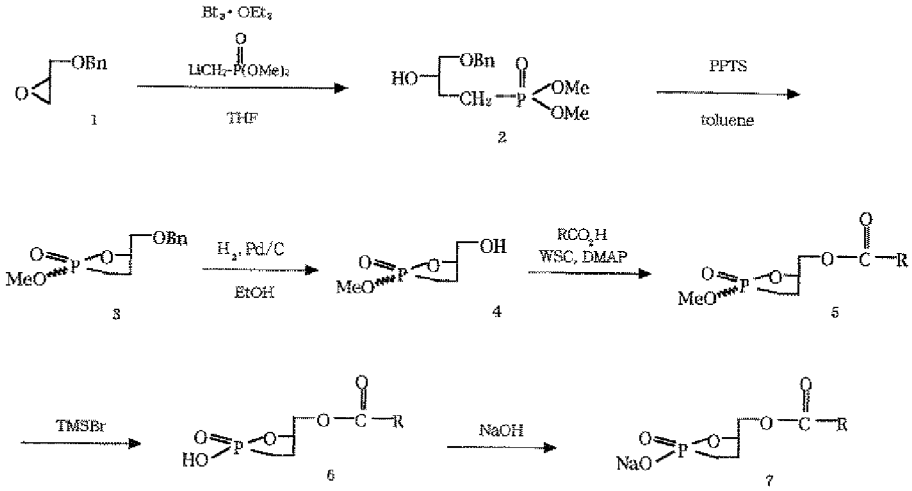


1 2 3 4 5 6 7

In the above formulae, at the outset, commercially available (R)-benzyl glycidyl ether (1) is activated with the aid of $BF_3 \cdot Et_2O$, n-BuLi is allowed to react with dimethyl methylphosphonate, and the resulting lithiated form is subjected to the reaction to obtain an alcohol (2).

The resulting alcohol is subjected to reaction in toluene with the use of an excessive amount of a pyridinium salt of p-toluenesulfonic acid at 80°C to obtain a cyclized form (3). The resulting cyclized form is hydrolyzed under a hydrogen atmosphere with the use of 20% $Pd(OH)_2$-C to perform debenzylation (4). 1-Ethyl-3-(3-dimethylaminopropyl)carbodiimide hydrochloride, as a condenser, is allowed to react with a fatty acid to obtain a coupled form (5). Subsequently, bromotrimethylsilane is used as a nucleophile to exclusively remove a methyl group in a position-selective manner, thereby obtaining a cyclic phosphonic acid (6). The resultant is introduced into a separatory funnel with the aid of ether, and a small amount of an aqueous solution of 0.02 N sodium hydroxide is added dropwise thereto to separate liquids. The compound of interest is extracted and purified as a sodium salt (7).

A compound represented by Formula (I) in which X represents a methylene group and Y represents an oxygen atom can be synthesized in accordance with the method described in JP Patent Publication (Kokai) No. 2004-010582 A or International Publication WO 2003/104246.

The therapeutic agent for pulmonary fibrosis according to the present invention is preferably provided in the form of a pharmaceutical composition that comprises one or more pharmaceutically acceptable additives and the compound represented by Formula (I) as an active ingredient.

The therapeutic agent for pulmonary fibrosis according to the present invention can be administered in various forms, and administration may be carried out orally or parenterally (for example, intravenous, intramuscular, subcutaneous or intracutaneous injection, rectal administration, and permucosal administration may be employed). Examples of dosage forms for pharmaceutical compositions suitable for oral administration include a tablet, a granule, a capsule, a powder, a solution, a suspension, and syrup. Examples of dosage forms for pharmaceutical compositions suitable for parenteral administration include an injection, an infusion, a suppository, and a percutaneous absorption agent. The dosage form for the agent of the present invention is not limited thereto. The therapeutic agent for pulmonary fibrosis of the present invention may also be an inhalation formulation. In the case of an inhalation formulation, the route of administration may be oral or nasal. Further, the agent can also be made into sustained-release formulations in accordance with methods known in the art.

Types of pharmaceutical additives used for producing the therapeutic agent for pulmonary fibrosis according to the present invention are not particularly limited, and a person skilled in the art can select adequate additives. Examples of additives that can be used include an excipient, a disintegration agent or a disintegration auxiliary agent, a binder, a lubricant, a coating agent, a base, a dissolving agent or a solubilizer, a dispersant, a suspension agent, an emulsifier, a buffer, an antioxidant, an antiseptic, an isotonic agent, a pH adjusting agent, a dissolving agent, and a stabilizer. Each specific ingredient used for the above purposes is well known to a person skilled in the art.

Examples of pharmaceutical additives that can be used for the production of oral preparations include: an excipient, such as glucose, lactose, D-mannitol, starch, or crystalline cellulose; a disintegration agent or a disintegration auxiliary agent, such as carboxymethyl cellulose, starch, or carboxymethyl cellulose calcium; a binder, such as hydroxypropyl cellulose, hydroxypropyl methylcellulose, polyvinyl pyrrolidone, or gelatin; a lubricant, such as magnesium stearate or talc; a coating agent, such as hydroxypropyl methylcellulose, white sugar, polyethylene glycol, or titanium oxide; and a base, such as Vaseline, liquid paraffin, polyethylene glycol, gelatin, kaolin, glycerin, purified water, or hard fat.

Examples of the pharmaceutical additives that can be used for production of an injection or an infusion preparation include: a dissolving agent or a solubilizer that can be used for an aqueous injection or a use-time dissolution

type injection, such as injection distilled water, physiological saline, propylene glycol, or a surfactant; an isotonic agent, such as glucose, sodium chloride, D-mannitol, or glycerin; and a pH adjusting agent, such as an inorganic acid, an organic acid, an inorganic base, or an organic base.

The therapeutic agent for pulmonary fibrosis according to the present invention can be administered to mammals, including humans.

A dose of the therapeutic agent for pulmonary fibrosis according to the present invention should be increased or decreased in accordance with conditions such as age, sex, body weight, symptoms of a patient, and the route of administration. The dose of the active ingredient per day per adult is generally 1 μg/kg to 1,000 mg/kg, and preferably 10 μg/kg to 100 mg/kg. The agent may be administered in the amounts mentioned above once a day or several separate times (for example, about 2-4 times) a day.

The present invention is described in greater detail with reference to the following examples, although the present invention is not limited to the examples.

Examples

<Materials and Methods>

(1) Test Substances and Media
(1-1) Test substance
2-Carbacyclic phosphatidic acid (2ccPA):

[Formula 4]

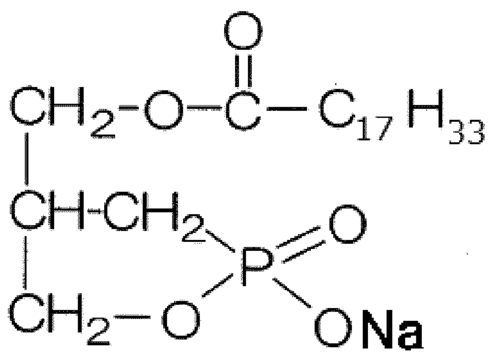

(1-2) Medium

Japanese Pharmacopoeia: Physiological saline solution (saline solution)

(2) Test system

Mouse Crl:CD1(ICR), 45 mice, male, purchased from Charles River Japan K.K. (11 weeks old at time of purchase)

(3) Preparation of dosing solution

The test substance was divided into 6 mg × 28 doses prior to the day of administration. Dose solution was prepared on the day of administration. To one bottle of the previously aliquoted test substance (6 mg), 6 mL of saline solution was added and mixed by inverting. The samples were heated (approximately 40°C) and sonicated until dissolution was visually confirmed (1 mg/mL solution). The 1 mg/mL solution was divided into 0.5 mL portions, and 4.5 mL of saline solution was added. Dissolution was confirmed by inverting and mixing (0.1 mg/mL solution).

(4) Administration

The route of administration was intraperitoneal administration.

For dosing acclimatization, the vehicle was administered intraperitoneally at a volume of 10 mL/kg once a day from Day -7 (the first day of administration of bleomycin and the test substance is Day 0) for a total of 7 days.
It was administered once per day for 28 days. Administration at the time of preparation of the pulmonary fibrosis model (Day 0 to Day 4) was before administration of bleomycin.
10 mL/kg was administered intraperitoneally using a 1 mL syringe and needle (Terumo Corporation).

(5) Test group composition

[Table 1]

| Test group number | Test group | Dosage (mg/kg) | Dosing volume (mL/kg) | number of starting case |
|---|---|---|---|---|
| 1 | Normal# | 0 | 10 | 8 |
| 2 | Control# | 0 | 10 | 10 |
| 3 | test substance low | 1 | 10 | 10 |
| 4 | test substance high | 10 | 10 | 10 |
| #: A vehicle was administered. | | | | |

(6) Experimental method

(6-1) Calculation method

Day 0 was defined as the first day of administration of the bleomycin solution and physiological saline.

(6-2) Body weight measurement

Measurements were taken every day from Day 0 to Day 4, and from Day -7 to Day -1 and from Day 5 onwards, measurements were taken 3 times/week (including at autopsy).

(6-3) Preparation of pulmonary fibrosis model

The bleomycin solution was administered into the tail vein of mice at a dose volume of 5 mL/kg (bleomycin dose: 15 mg/kg) once a day for 5 days. The volume of administered liquid was calculated based on the latest body weight and calculated to the nearest 0.01 mL (rounded to the third decimal place). The normal group was administered physiological saline at a volume of 5 mL/kg.

(6-4) Blood collection

Blood collection was carried out on the day after the final administration (Day 28). Blood collection was performed for all survivors. The animals were subjected to laparotomy under 2% isoflurane anesthesia, and whole blood was collected from the abdominal vena cava using a heparin-added syringe and a syringe needle. After that, the abdominal aorta was incised to kill the animal by exsanguination. Plasma was collected from the collected blood using a centrifuge with centrifugation conditions set at 4°C, 1800g and 10 minutes. Plasma was dispensed into approximately 100 μL × 2 tubes and a total of 3 tubes and was stored in an ultra-low temperature freezer (permissible range: -90 to -65°C).

(6-5) Collection of lungs, measurement of right lung weight, and fixation

After lethal exsanguination, the right bronchial bifurcation was ligated, and only the right lung was removed. The wet weight of the excised right lung was measured. After weighing, the right lung was minced. After that, the right lung was dried in a dry heat sterilizer set at 72°C for about 72 hours, and the dry weight of the right lung was measured. After measuring the dry weight, it was stored in an ultra-low temperature freezer (permissible range: -90 to -65°C) until the amount of hydroxyproline was measured. The left lung was ligated and fixed after a cannula was inserted into the trachea, and a 10% phosphate-buffered formalin solution was injected through the cannula at a pressure of 15cm $H_2O$ to expand and fix the lung tissue. Lung sampling was performed on animals from which blood collection was performed.

A fixed left lung is undergoing histopathological examination.

(6-6) Measurement of Hydroxyproline (HYP) in the right lung

Preparation of sample for hydroxyproline measurement

The hydroxyproline content of the right lung cryopreserved in section (6-5) was measured. 2 mL of HCl for hydrolysis was added to the dried right lung and the sample was hydrolyzed in an autoclave at about 121°C for 30 minutes. After that, it was neutralized with 2.5 N NaOH (pH about 6 to 7: pH test paper was used for confirmation). By measuring the tare weight in advance and measuring the weight after neutralization, the amount (g) of the sample after neutralization was calculated. The sample after neutralization was appropriately diluted with water for injection, and 2 mL of the solution was used as a sample for measurement of HYP content.

Creation of calibration curve

1 mL of chloramine T reagent was added to 2 mL of the HYP standard solution and the mixture was left stand at room temperature for 20 minutes. Then, 1 mL of perchloric acid reagent was added and the mixture was left stand at room temperature for 5 minutes. After adding 1 mL of p-dimethylaminobenzaldehyde reagent and heating at 60 °C for 20 minutes, the absorbance at 557 nm of the supernatant which was obtained by filtration using a filter was measured with a spectrophotometer to create a calibration curve.

Measurement of HYP content in samples

For 2 mL of each sample for measurement of HYP content, the absorbance was measured in the same manner as described in Section 3.6.6.2, and the HYP content (μg/mL) was calculated from the measured values based on the calibration curve obtained in Section 3.6.6.2. Each sample for measurement of HYP content was diluted 20-fold and was measured. Single measurement was used.

$$\text{Amount of HYP } (\mu\text{g/mL}) \times \text{Dilution ratio} \times \text{Amount of sample after neutralization (g)} = \text{Amount of HYP in the right lung } (\mu\text{g/Lung})$$

(7) Statistical analysis (USOP/STA/1001)

(7-1) Processing items

Right lung wet weight, right lung dry weight, amount of HYP in right lung, and left lung histopathological examination (if performed)

(7-2) Processing method

The test with the following combinations is carried out.

Right lung wet weight, right lung dry weight and amount of HYP in right lung
Student's t-test: study group number 1 vs 2, study group number 2 vs 3, study group number 2 vs 4 Dunnett test: test group number 2 vs 3, 4
Histopathological examination (if performed)
Wilcoxon test: test group number 1 vs 2
Steel test: test group number 2 vs 3, 4
The significance level for each is 5%, and the results are divided into 1 and 5%.

(7-3) Analysis software

SAS 9.4[EXSUS Version 8.1.0, SAS Institute Japan Corporation (CAC Chlore Corporation)] is used.

<Results>

Right lung weight and hydroxyproline amount were shown in Figs. 1 to 3 and Table 2.

In Figs 1 to 3, each value represents mean ± S.E.M.

##: p <0.01; significantly different from the Normal group by Student's t test

**: p <0.01; significantly different from Control group by Student's t test

*: p <0.05; significantly different from Control group by Student's t-test

[Table 2]

Table 2: Effect of 2ccPA on wet and dry weights and hydroxyproline of the right lung of bleomycin-induced pulmonary fibrosis model mice

| Groups | Dose (mg/kg) | N | | Wet weight of right lung (g) | | | | Dry weigt of right lung (g) | | | | Hydr oxypr oline (μ g/lung) | | | |
|---|---|---|---|---|---|---|---|---|---|---|---|---|---|---|---|
| Normal | 0 | 8 | | 0.1224 | ± | 0.0029 | | 0.0236 | ± | 0.0007 | | 266.9 | ± | 6.4 | |
| Control | 0 | 10 | | 0.1735 | ± | 0.0087 | ## | 0.0348 | ± | 0.0017 | ## | 354.9 | ± | 15.1 | ## |
| | | | vs Normal | P=0.0001 | | | | P=0.0000 | | | | P=0.0002 | | | |
| 2ccPA | 1 | 9 | | 0.1599 | ± | 0.0113 | | 0.0327 | ± | 0.0021 | | 331.2 | ± | 20.0 | |
| | | | vs Control | P=0.3487 | | | | P=4550 | | | | P=0.3513 | | | |
| 2ccPA | 10 | 9 | | 0.1458 | ± | 0.0044 | | 0.0303 | ± | 0.0011 | | 288.8 | ± | 11.1 | |
| | | | vs Control | P=0.0141 | | | ** | P=0.0480 | | | * | P=0.0029 | | | ** |

Each value represents mean ± S.E.M.

##: $p < 0.01$; significantly different from the Normal group by Student's t test

**: $p < 0.01$; significantly different from Control group by Student's t test

*: $p < 0.05$; significantly different from Control group by Student's t-test

## Claims

1. A therapeutic agent for pulmonary fibrosis which comprises, as an active ingredient, a compound represented by the following formula (I):

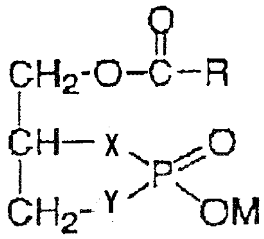

(I)

wherein R represents a linear or branched alkyl group having 1 to 30 carbon atoms, a linear or branched alkenyl group having 2 to 30 carbon atoms, or a linear or branched alkynyl group having 2 to 30 carbon atoms, which may contain a cycloalkane or aromatic ring; X and Y each independently represent an oxygen atom or a methylene group, provide that X and Y do not simultaneously represent a methylene group; and M represents a hydrogen atom or an alkali metal atom.

2. The therapeutic agent for pulmonary fibrosis according to claim 1, wherein, in Formula (I), either X or Y represents an oxygen atom and the other represents a methylene group.

3. The therapeutic agent for pulmonary fibrosis according to claim 1 or 2, wherein the compound represented by Formula (I) is carbacyclic phosphatidic acid of 1-oleoyl-cyclic phosphatidic acid, 1-palmitoleoyl-cyclic phosphatidic acid, or a derivative thereof.

[Fig. 1]

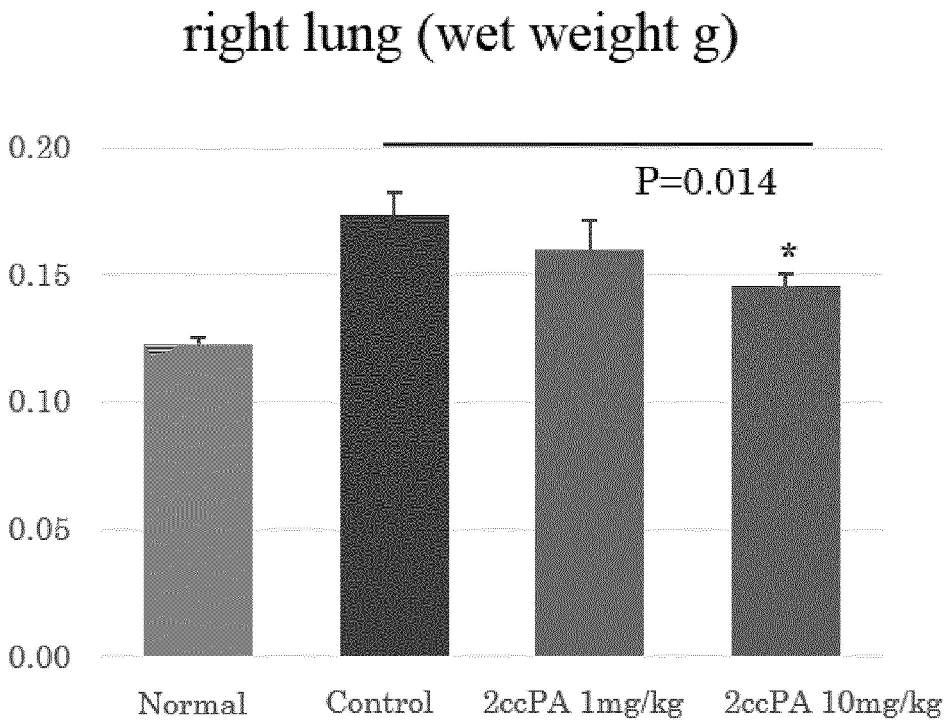
right lung (wet weight g)
0.20
0.15
0.10
0.05
0.00
P=0.014
*
Normal
Control
2ccPA 1mg/kg
2ccPA 10mg/kg

[Fig. 2]

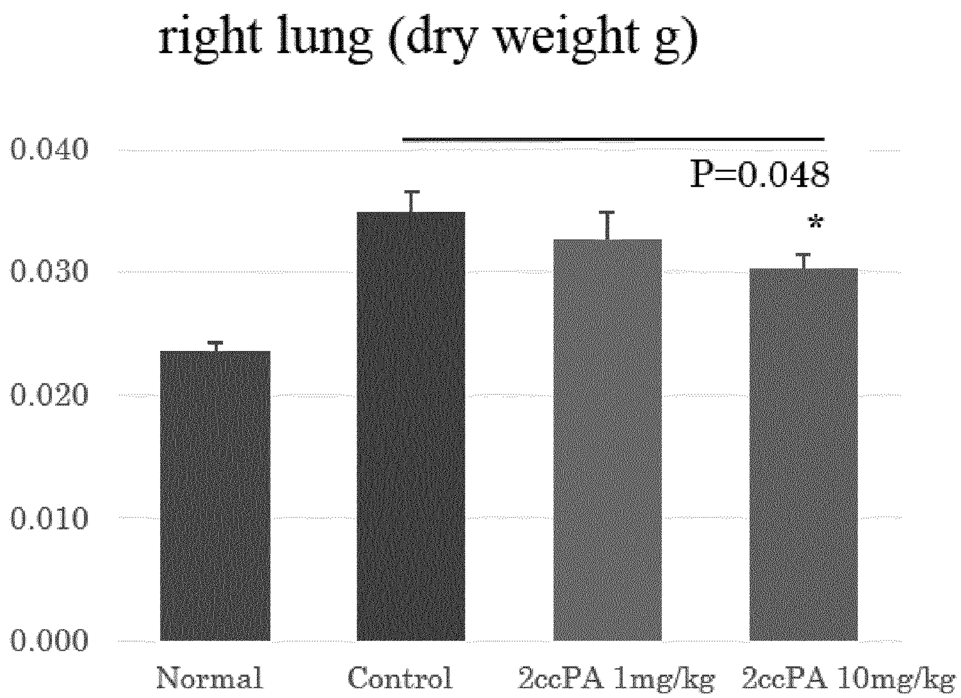
right lung (dry weight g)
0.040
0.030
0.020
0.010
0.000
P=0.048
*
Normal
Control
2ccPA 1mg/kg
2ccPA 10mg/kg

[Fig. 3]

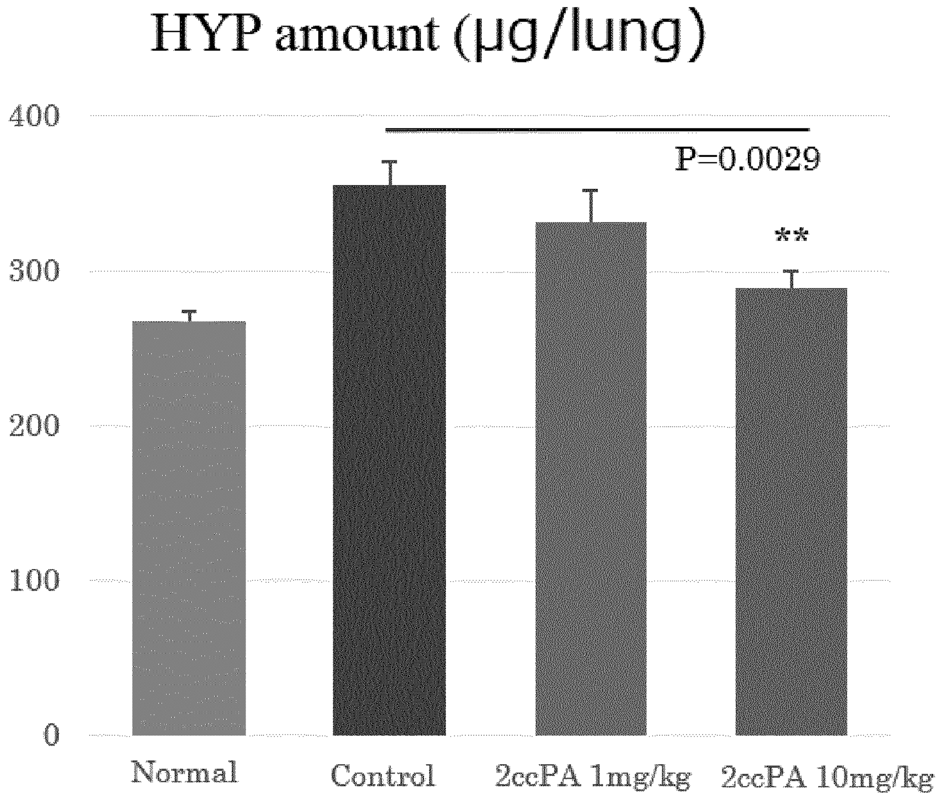
HYP amount (µg/lung)
400
300
200
100
0
P=0.0029
**
Normal
Control
2ccPA 1mg/kg
2ccPA 10mg/kg

## INTERNATIONAL SEARCH REPORT

International application No. **PCT/JP2021/043186**

**A. CLASSIFICATION OF SUBJECT MATTER**

***A61K 31/683***(2006.01)i; ***A61P 11/00***(2006.01)i; ***A61P 43/00***(2006.01)i
FI: A61K31/683; A61P11/00; A61P43/00 105

According to International Patent Classification (IPC) or to both national classification and IPC

**B. FIELDS SEARCHED**

Minimum documentation searched (classification system followed by classification symbols)

A61K31/683; A61P11/00; A61P43/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY/MEDLINE/EMBASE/BIOSIS (STN), JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| Y | HIGUCHI, T. et al. 2-Carba cyclic phosphatidic acid (2ccPA) suppresses profibrotic activity in Systemic sclerosis skin fibroblasts and bleomycin-induced skin fibrosis in mice. Annals of the Rheumatic Diseases. 2019, vol. 78, Supplement 2, pages 447, abstract Number: THU0333<br>columns of background, results, conclusion | 1-3 |
| Y | TIGYI, G. Aiming drug discovery at lysophosphatidic acid targets. British Journal of Pharmacology. 2010, vol. 161, no. 2, pages 241-270<br>page 243, left column, third paragraph, page 256, left column, second paragraph | 1-3 |
| Y | TAGER, A. M. et al. The lysophosphatidic acid receptor LPA1 links pulmonary fibrosis to lung injury by mediating fibroblast recruitment and vascular leak. Nat. Med. 2008, vol. 14, pages 45-54<br>abstract, fig. 2 | 1-3 |
| A | HIGUCHI, T. et al. Antifibrotic effects of 2-carba cyclic phosphatidic acid (2ccPA) in systemic sclerosis: contribution to the novel treatment. Arthritis research & therapy. 2019, vol. 21, no. 1, 103, pages 1-10<br>entire text | 1-3 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 January 2022** | **25 January 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

INTERNATIONAL SEARCH REPORT

International application No.
**PCT/JP2021/043186**

**C. DOCUMENTS CONSIDERED TO BE RELEVANT**

| Category* | Citation of document, with indication, where appropriate, of the relevant passages | Relevant to claim No. |
|---|---|---|
| A | JP 2008-208058 A (NOF CORP.) 11 September 2008 (2008-09-11) | 1-3 |

Form PCT/ISA/210 (second sheet) (January 2015)

**INTERNATIONAL SEARCH REPORT**
**Information on patent family members**

International application No.
**PCT/JP2021/043186**

| Patent document cited in search report | Publication date (day/month/year) | Patent family member(s) | Publication date (day/month/year) |
|---|---|---|---|
| JP 2008-208058 A | 11 September 2008 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5465815 B
- JP 2012056853 A
- JP 4163007 B
- JP 6736466 B
- JP H05230088 A
- JP H07149772 A
- JP H07258278 A
- JP H0925235 A
- JP 2001178489 A
- JP H11367032 A
- WO 2002094286 A
- JP 2004010582 A
- WO 2003104246 A


### Non-patent literature cited in the description


- **KOBAYASHI, S. et al.** *Tetrahedron Letters,* 1993, vol. 34, 4047-4050